# FASCICULE DE BREVET EUROPEEN

(11) **EP 2 349 485 B1**
(45) Date de publication et mention de la délivrance du brevet: **29.06.2016**
(21) Numéro de dépôt: 09743913.7
(22) Date de dépôt: 21.10.2009
(51) Int. Cl.: A61P 31/00, A61K 38/16

(54) **MÉDICAMENT COMPRENANT DES GLYCOPROTÉINES DE KLEBSIELLA PNEUMONIAE DESTINÉ À TRAITER DES INFECTIONS**
ARZNEIMITTEL ENTHALTEND GLYKOPROTEINE VON KLEBSIELLA PNEUMONIAE ZUR BEHANDLUNG VON INFEKTIONEN
DRUG COMPRISING GLYCOPROTEINS OF KLEBSIELLA PNEUMONIAE FOR TREATING INFECTIONS

(30) Priorité: 31.10.2008 CH 17072008
(43) Date de publication de la demande: 03.08.2011
(73) Titulaire: Etienne, Marie-Christine, 1227 Genève (CH)
(72) Inventeur: Etienne, Marie-Christine, 1227 Genève (CH)
(74) Mandataire: KATZAROV S.A.
(86) Numéro de dépôt international: PCT/IB2009/007201
(87) Numéro de publication internationale: WO 2010/049776

(56) Documents cités:
- FR-A1- 2 462 477
- FR-A1- 2 490 495
- FR-A1- 2 674 755
- FR-A1- 2 842 208
- SMETS P ET AL: "RU-41740 (K. Pneumoniae Glycoprotein) enhances resistance to experimental candidiasis and stimulates phagocytic functions" ANNALES DE L'INSTITUT PASTEUR. IMMUNOLOGIE, ELSEVIER, PARIS, FR, vol. 138, no. 3, 1 janvier 1987 (1987-01-01), pages 425-436, XP025862058 ISSN: 0769-2625 [extrait le 1987-01-01]
- NIMIER K ET AL: "Protective effects of RU 41740, a bacterial immunomodulator, against experimental infections: induction of cytokine and immunoglobulin release in mice after oral administration." INTERNATIONAL JOURNAL OF IMMUNOPHARMACOLOGY SEP 1999, vol. 21, no. 9, septembre 1999 (1999-09), pages 561-574, XP002572716 ISSN: 0192-0561

## Description

La présente invention concerne l'utilisation de glycoprotéines extraites de la bactérie klebsiella Pneumoniae pour la fabrication d'un médicament destiné au traitement d'infections aiguës ou d'infections chroniques.

Il s'agit de glycoprotéines extraites de Klebsiella Pneumoniae, caractérisées en ce qu'elles sont retenues sur membrane de porosité moyenne 0,011 micromètre, à la dose de 1 mg exprimée en produit anhydre ,par comprimé, sachet, gélule, ou tout autre vecteur thérapeutique.

Il s'agit de fractions membranaires de Klebsiella Pneumoniae caractérisées en ce qu'elles sont des protéoglycanes de Klebsiella Pneumoniae qui se trouvent à la dose de 1,125 mg par comprimé , ou sachet , ou gélule ou tout autre vecteur thérapeutique.

Ces protéoglycanes membranaires peuvent être utilisés seuls ou associés à des ribosomes bactériens titrés à 70% d'ARN caractérisé en ce que pour 1,125 mg de protéoglycanes membranaires , il y a 0,750 mg de ribosomes bactériens , se décomposant comme suit :0,2625 mg de ribosomes de Klebsiella Pneumoniae , 0,225 mg de ribosomes de Streptococcus Pneumoniae , 0,225 mg de ribosomes de Streptococcus Pyogènes du groupe A , 0,0375 mg de ribosomes de Haemophilus Influenzae.

Vu la faible teneur en produit actif ces glycoprotéines sont accompagnées , au sein du médicament d'excipients destinés à favoriser leur bonne diffusion dans l'organisme: On utilisera pour la fabrication d'un comprimé de préférence un excipient tel que défini ci après et comprenant :Lactose, amidon de maïs, stéarate de magnésium, acéthylphtalate de cellulose, dibuthylphtalate, saccharose, carmellose sodique silice colloïdale ,talc , polysorbate 80 ,dioxyde de titane cire blanche.

On utilisera également pour la fabrication d'un comprimé ,de préférence un autre type d'excipient comprenant: aérosil , stéarate de magnésium, et sorbitol.

Tout autre type d'excipient favorisant la diffusion des glycoprotéines pourra être utilisé.

L'invention est caractérisée en ce que ces glycoprotéines ainsi définies plus haut sont utilisées à la dose de deux comprimés par jour: à raison de un comprimé matin et soir pour un individu pesant jusqu'à 50 kg ; au delà , il est nécessaire d'augmenter un peu les doses si on ne veut pas avoir un effet atténué. Il est possible de diminuer ces doses ou de les augmenter, de façon à adapter le traitement à la situation pathologique.

Dans l'art antérieur, ces glycoprotéines extraites de Klebsiella Pneumoniae telles que décrites plus haut ont été commercialisées sous le nom de Biostim et Ribomunyl en France. Dans l'art antériéur, elles étaient utilisées comme traitement préventif des infections récidivantes de la sphère ORL et des bronches, indication pour laquelle ces produits avaient obtenu une AMM.

Il était connu dans l'art antérieur que ces faibles doses de glycoprotéines étaient capables de renforcer les défenses immunitaires surtout si jugées déficientes, et d'augmenter la résistance de l'organisme à des infections survenant postérieurement à leur priser. On les prenait sur plusieurs semaines espacées de fenêtres thérapeutique.

L'invention concerne une propriété surprenante et inattendue de ces glycoprotéines, à savoir que prises au début d'une infection , ces glycoprotéines provoquent , au moyen d'une très forte stimulation de tous les systèmes de défense antiinfectieux de l'organisme, de courte durée :12 à 24 heures heures maximum ,une lyse rapide des agents pathogènes de toute nature et par là ,une guérison de l'infection. (ce qui nécessite le renouvellement des prises toutes les 12 heures) Ces glycoprotéines provoquent , dans ces conditions , une forte stimulation de tout le système immunitaire , avec forte augmentation de toutes les cellules immunocompétentes , forte augmentation des cellules destinées à détruire les agents pathogènes, forte augmentation de tous les anticorps dont les IgM, les IgG et les IgA destinés à détruire les agents pathogènes. Elles augmentent aussi tous les médiateurs anti infectieux. Tous les agents pathogènes quelle que soit leur nature peuvent ainsi être détruits par l'action de ces glycoprotéines: bactéries, virus, mycoses, et autres: bacille, prion.

Dans l'art antérieur , il est nécessaire, pour traiter des infections d'employer des produits adaptés à l'agent pathogène et au site de l'infection: Antibiotique pour les bactéries, antivirus pour les virus , antifungique pour les mycoses. Utilisés à des doses 100 ou 1000 fois supérieures à celles des glycoprotéines: 2 Mg par jour, ces produits occasionnent fatigue et réactions d'intolérance , ce qui n'est pas le cas avec ces glycoprotéines qui ne donnent pas l'impression d'avoir pris un remède et qui ont donc une tolérance exceptionnelle.

D'une façon surprenante et inattendue, ces glycoprotéines ont fait la preuve de guérir un nombre important d'infections aiguës très courantes sans caractère de gravité immédiat, spécialement d'infections ORL et d'infections chroniques, particulièrement d'infections chroniques persistant après traitement antibiotique.

Citons des infections aiguês qui sont particulièrement concernées par l'invention et sont de préférence:
_ les infections ORL: rhinites, rhinopharyngites, amygdalites, otites, sinusites etc...
_ Les infections digestives: gastro entérites , infections gastriques , intestinales ,etc...
_ Les infections respiratoires :laryngites , trachéites , bronchites , etc...
_ Les infections génito urinaires: cystites , vaginites, les infections pelviennes etc...

L'invention concerne toutes les infections chroniques sans limitation et tout particulièrement les infections chroniques persistant après traitement antibiotique , particulièrement les otites chroniques.

Si la plupart du temps, ces glycoprotéines utilisées seules ont fait la preuve de leur efficacité dans ce type d'infections aiguës ou d'infections chroniques, l'association à d'autres extraits bactériens peut potentialiser leur action. Ces extraits peuvent être des ribosomes bactériens ou des lysats bactériens antigéniques appartenant à diverses préparations telles que :IRS 19 , Rhinopten , Imudon , Lantigen B , Amphovaccin intestinal ou urinaire etc...

L'invention concerne particulièrement les maladies respiratoires contagieuses qui se produisent le plus souvent en hiver, qui sont dues en partie à des virus , en partie à des bactéries et qui commencent par une rhinopharyngite fébrile et évoluent vers une bronchite sur 10 jours en occasionnant de la température sur toute l'évolution

Ce traitement par glycoprotéines extraites de Klebsiella Pneumoniae a été efficace pour guérir en 2 à 3 jours un nombre important de ces affections respiratoires au stade de rhinopharyngite, en évitant ainsi l'évolution qui se faisait régulièrement auparavant vers la bronchite.

Ce traitement par glycoprotéines selon l'invention a été efficace pour guérir un nombre important de catarrhe de la trompe d'Eustache, ainsi qu'un nombre important d'otites congestives.

Ce traitement par glycoprotéines selon l'invention a guéri une otite chronique qui persistait après 3 semaines d' antibiotiques avec température persistante , douleur d'oreille sourde, et apparition récente d'un acouphène témoignant de la progression de l'infection à l'organe de l'audition qui se trouve dans la mastoïde-qui était douloureuse. Il a fallu un mois de traitement par ces glycoprotéines pour guérir tous les symptômes. L'acouphène avait disparu dans les premiers jours de ce traitement. La posologie était , comme toujours de un comprimé matin et soir. Il y a eu des fenêtres thérapeutiques de un jour chaque semaine.

Le traitement de glycoprotéines extraites de Klebsiella Pneumomae a été efficace pour guérir des atteintes de conjonctivite avec une suppuration conjonctivale très importantes, résistant à des collyres antibiotiques et antiseptiques, faisant craindre une évolution grave vers une kératite infectieuse ou une infection profonde de l'oeil avec risque de perte de l'oeil. Dès la première prise de glycoprotéine il y a eu une diminution de la quantité de pus conjonctival, une disparition d'un état subfébrile associé d'une rhinite associée. Il a fallu 8 jours de traitement par ces glycoprotéines pour guérir complètement ces conjonctivites. L'allure clinique évoquait une origine virale de ces conjonctivites.: adénovirus, VRS.

Plusieurs atteintes d'orgelets ont guéries en 3 à 5 jours avec traitement par ces glycoprotéines .

Plusieurs atteintes d'aphtes buccaux ont réagies à ce traitement par ces glycoprotéines selon l'invention avec dans les heures qui ont suivi la première prise de glycoprotéine une diminution spectaculaire des aphtes en question Le traitement a duré 3 à 4 jours.

Plusieurs atteintes d'infections des conduits auditifs externes ont été traitées avec succès par ces glycoprotéines selon l'invention, avec dès la première prise de ces glycoprotéines , une diminution nette des douleurs d'oreille du prurit , des signes associés comme une petite température ou une très légère gêne auditive. Il a fallu 5 à 6 jours de traitement
Ces glycoprotéines extraites de Klebsiella Pneumoniae se sont révélées efficaces pour guérir des gastroentérites bénignes parmi lesquelles il y avait peut-être des infections à Helico Bacter Pylori . Dès la première prise de ces glycoprotéines, il y a eu une disparition nette des nausées et des vomissements quand il y en avait, un meilleur état général: une disparition de la fatigue, de la petite température associée quand il y en avait, une cédation de symptômes associés quand il y en avait: rhinite , céphalées , diarrhée. Ce traitement a duré 2 jours en moyenne.

Ce traitement de glycoprotéines selon l'invention a été efficace pour guérir des douleurs abdominales persistantes après diverses infections :digestives ou gynécologiques après traitement antibiotique telles que sigmoïdite ou chlamydioses pelviennes . Il a fallu une semaine ou deux de traitement par ces glycoprotéines.

Ce traitement par glycoprotéines extraites de Klebsiella Pneumoniae s'est avéré efficace pour faire passer des signes persistants de cystites après traitements antibiotiques tels que pollakiurie, brûlures en urinant , et pour juguler des début de rechutes de ces cystites avec les mêmes symptômes.

Ces glycoprotéines ont prouvé leur efficacité dans des infections virales telles que des atteintes de zona (2) où l'éruption a disparu en 3 jours avec un traitement par ces glycoprotéines a vec , dès la première prise de ces glycoprotéines disparition d'un état subfébrile et meilleur état général.

Un traitement par ces glycoprotéines selon l'invention a guéri une atteinte de toux quinteuse persistante et résistante à un traitement antibiotique et qui était survenue après un contact avec un enfant atteint de coqueluche.

Un traitement par 8 jours de ces glycoprotéines a coïncidé avec la disparition d'un papillomavirus cancérigène constaté sur un frottis et qui n' y était plus sur le frottis suivant Si on ne peut rien conclure sur une atteinte on peut néanmoins penser que ces glycoprotéines sont capables de faire éliminer par l'organisme les virus cancérigènes et les autres mincroorganismes responsables de cancer tels que l'Helicobacter Pylori.

Ces glycoprotéines extraites de Klebsiella Pneumoniae se sont avérées efficaces, en cas de contact avec un malade contagieux , pour éviter que ne se déclare une maladie: Un jour de traitement ou deux ont été nécessaire.

Plusieurs atteintes de toux avec expectorations persistantes plus ou moins longtemps après traitement antibiotique ont été été guéries par la prise de ces glycoprotéines pendant 8 jours en moyenne . L'une des atteintes évoquait fortement une infection par le virus respiratoire syncitial.

Il y a eu 8% d'échecs au traitement par ces glycoprotéines. Il s'est agi d'atteintes de rhinopharyngites qui ont évoluées vers une bronchite malgré ce traitement de glycoprotéines. Il y a eu alors une accentuation de la réaction inflamatoire bronchique avec petite augmentation du volume des crachats. Il se peut vraissemblablement que la grande virulence de l'agent infectieux ait mis en échec la capacité de résistance de l'organisme et la mobilisation de tous les anticorps Il se peut aussi que la raison de l'échec soit l'absence de contact antérieur avec l'agent infectieux ce qu'il fait qu'il n'y avait pas de cellules à mémoire à mobiliser. Dans les échecs, le traitement antibiotique a été très écourté par rapport à ce qu'il aurait été sans traitement préalable par ces glycoprotéines: Il a duré deux jours en moyenne.

On peut donc conclure que les glycoprotéines extraites de la bactérie Klebsiella Pneumoniae sont très efficaces pour le traitement d'infections qui n'ont pas de caractère de gravité immédiate cad en l'absence de pouvoir pathogène intense des agents infectieux . On peut conclure que ces glycoprotéines sont efficaces pour le traitement des infections débutantes de là sphère ORL, qui sont très fréquentes en hiver, ainsi que pour le traitement des infections chroniques persistant après antibiotiques, notament pour le traitement des otites chroniques qui , dans l'art antérieur évoluent presque toujours vers des pertes graves de l'audition.

On peut donc conclure aussi que cette nouvelle utilisation de glycoprotéines extraites de Klebsiella Pneumoniae est très prometteuse en matière d'applications industrielles et de commercialisation.
En effet, ce sont les produits les mieux tolérées en raison de leur faible dose de substance active. Ils sont susceptibles de convenir à 96% de la population, pour un usage fréquent. En faisant détruire les agents pathogènes , leur premier effet est de faire tomber la fièvre .Dans les cas d'échecs sur les survenues de bronchites , ils ont toujours évité la survenue d'otites.
Les 4% de la population qui ne bénéficieront pas de ce nouvel usage de ces glycoprotéines sont ceux qui sont porteurs de contrindications: En augmentant le taux de tous les anticorps , ces glycoprotéines augmentent aussi celui des anticorps responsables de l'allergie et de l'auto immunité. (IgE pour les allergies)Les allergies bénignes comme des rhinites allergiques ou des eczémas limités ne sont pas des contrindications: leur aggravation est réversible avec retour à l'état initial 24 heures après arrêt de ces glycoprotéines . Les allergies graves comme l'asthme sont des contrindications à l'emploi de ces glycoprotéines . Il faut être prudent chez les sujets qui ont un terrain allergique et qui sont porteurs de prothèses diverses ou de cristallins artificiels .Les infections virales à rétrovirus doivent inciter à la prudence car les rétrovirus infectent les cellules immunocompétentes et qu'en faisant multiplier ce type de cellules, on a des chances de faire multiplier des cellules infectées et donc les virus. Mais cette hypothèse n'a pas été étudiée lors des essais . Ces infections à rétrovirus sont le SIDA les Lymphosarcomes , les lymphomes Toutes les leucémies et leucoses sont des contrindications dans dans cette optique.

L'industrie pharmaceutique a renoncé à l'utilisation de ces glycoprotéines extraites de Klebsiella Pneumoniae en les retirant toutes du marché en 2006 , dans l'ignorance des propriétés surprenantes et inattendues de ces glycoprotéines, objet de la présente invention. Cette industrie a préférré se recentrer et conserver des produits destinés à faire tomber la fièvre, comme l'aspirine et le paracétamol et des produits destinés à détruire les agents pathogènes tels que les antibiotiques et les antiviraux , sans soupçonner un instant que ces glycoprotéines pouvaient avoir ces propriétés.

## Revendications

1. Utilisation de glycoprotéines extraites de la bactérie Klebsiella Pneumoniae pour la fabrication d'un médicament destiné au traitement curatif des infections aiguës et des infections chroniques, particulièrement quand ces infections persistent après un traitement antibiotique, **caractérisée en ce que** la glycoprotéine est retenue sur membrane de porosité moyenne 0,011 micromètre et est à la dose de 1 mg par comprimé ou sachet ou de protéoglycanes membranaires extraites de la bactérie Klebsiella Pneumoniae à la dose de 1,125 mg par comprimé ou gélule, pour une posologie moyenne de 2 doses par jour.

2. Utilisation selon la revendication 1, **caractérisée en ce que** ces glycoprotéines sont utilisées seules au sein d'un médicament, ou associées soit à des lysats antigéniques de diverses bactéries en proportion variables, soit à des ribosomes de diverses bactéries titrés à 70% d'ARN , de compositions variables et de proportions variables.

3. Utilisation selon les revendications 1 et 2, **caractérisées en ce que** ces glycoprotéines ont toutes la même formule chimique ou ont des formules chimiques différentes.

4. Utilisation selon les revendications 1 et 3 **caractérisées en ce que** ces glycoprotéines provoquent, au moyen d'une très forte stimulation de tout le système immunitaire, une forte augmentation de toutes les cellules immunocompétentes, une forte augmentation des cellules destinées à détruire les agents pathogènes, une forte augmentation de tous les anticorps spécialement des IgM, IgG et des IgA, ainsi que des médiateurs de défense anti-infectieuse, et que cette forte stimulation du système immunitaire est de courte durée, n'excédant pas 12 à 24 heures, ce qui nécessite de renouveler les prises de médicament les renfermant toutes les 12 heures.

5. Utilisation selon les revendications 1 et 3 **caractérisée en ce que** le médicament est destiné à traiter des infections aiguës spécialement quand les organes concernés sont de la sphère ORL pulmonaire, ophtalmologique, intestinale, urinaire, particulièrement quand ces infections débutent, et tout spécialement quand l'infection est une otite ou une autre infection respiratoire commençant par une rhinopharyngite.

6. Utilisation selon les revendications 1 et 3, **caractérisée en ce que** le médicament est destiné à traiter des infections chroniques particulièrement quand les organes concernés sont des sphères ORL, pulmonaire , intestinale, génitourinaire, et tout spécialement quand l'infection est une otite chronique.

## Patentansprüche

1. Verwendung von aus dem Bakterium *Klebsiella pneumoniae* gewonnenen Glykoproteinen zur Herstellung eines Medikaments für die heilende Behandlung von akuten Infektionen und chronischen Infektionen, insbesondere wenn diese Infektionen nach einer antibiotischen Behandlung fortbestehen, **dadurch gekennzeichnet, dass** das Glykoprotein über eine Membran mit einer mittleren Porosität von 0.011 Mikrometer gehalten wird und bei einer Dosis von 1mg pro Tablette oder Sachet ist oder von aus dem Bakterium *Klebsiella pneumoniae* gewonnenen membranären Proteoglykane bei einer Dosis von 1.125mg pro Tablette oder Kapsel ist, für eine mittlere Dosierung von 2 täglichen Dosen.

2. Verwendung gemäss Anspruch 1, **dadurch gekennzeichnet, dass** diese Glykoproteine alleine innerhalb eines Medikaments verwendet werden, oder in Verbindung entweder mit Antigenlysaten aus verschiedenen Bakterien in veränderlichem Verhältnis oder mit zu 70% RNA titrierten Ribosomen aus verschiedenen Bakterien, in verschiedenen Kompositionen und in veränderlichem Verhältnis verwendet werden.

3. Verwendung gemäss Ansprüchen 1 und 2, **dadurch gekennzeichnet, dass** diese Glykoproteine alle die gleiche chemische Formel oder verschiedene chemische Formeln aufweisen.

4. Verwendung gemäss Ansprüchen 1 und 3, **dadurch gekennzeichnet, dass** diese Glykoproteine mittels einer sehr starken Stimulierung des gesamten Immunsystems eine starke Zunahme aller immunkompetenten Zellen, eine starke Zunahme der für die Vernichtung von pathogenen Agenten bestimmten Zellen, eine starke Zunahme aller Antikörper speziell IgM, IgG und IgA, sowie der antiinfektiösen Schutzmediatoren verursachen, und dass diese starke Stimulierung des Immunsystems von kurzer Dauer ist und 12 bis 24 Stunden nicht übersteigt, was die erneute Aufnahme der sie enthaltenden Medikamente alle 12 Stunden erfordert.

5. Verwendung gemäss Ansprüchen 1 und 3, **dadurch gekennzeichnet, dass** das Medikament zur Behandlung von akuten Infektionen bestimmt ist, insbesondere wenn die betroffenen Organen aus dem Bereich Lungen, Augen, Darm, Harnapparat stammen, insbesondere wenn diese Infektionen beginnen und ganz besonders, wenn die Infektion eine Ohreninfektion oder eine andere mit einer Nasopharyngitis beginnende Infektion der Atemwege ist.

6. Verwendung gemäss Ansprüchen 1 und 3, **dadurch gekennzeichnet, dass** das Medikament zur Behandlung von chronischen Infektionen bestimmt ist, insbesondere wenn die betroffenen Organe aus dem Bereich HNO, Lungen, Darm, Urogenitaltrakt stammen und insbesondere wenn die Infektion eine chronische Ohreninfektion ist.

## Claims

1. Use of glycoproteins extracted from the bacteria *Klebsiella pneumoniae* for producing a drug designed for the curative treatment of acute infections and chronic infections, in particular when these infections persist after an antibiotic treatment, **characterized in that** the glycoprotein is held on membrane of average porosity of 0.011 micrometres and is at the dose of 1mg per tablet or sachet, or of membrane proteoglycans extracted from the bacteria *Klebsiella pneumoniae* at the dose of 1.125mg per tablet or capsule, for an average dosage of 2 doses daily.

2. Use according to claim 1, **characterized in that** these glycoproteins are used alone within a drug or associated either with antigenic lysates of various bacteria in variable proportions or with ribosomes from various bacteria titrated at 70% RNA, of variable compositions and in variable proportions.

3. Use according to claims 1 and 2, **characterized in that** these glycoproteins have all the same chemical formula or have different chemical formulas.

4. Use according to claims 1 and 3, **characterized in that** these glycoproteins provoke, by means of a very strong stimulation of the entire immune system, a strong increase of all immune-competent cells, a strong increase of the cells designed for destroying pathogen agents, a strong increase of all the antibodies in particular IgM, IgG and IgA, as well as antiinfection defence mediators, and that this strong stimulation of the immune system is of short duration, not exceeding 12 to 24 hours, which requires the intake of the drug containing them to be renewed every 12 hours.

5. Use according to claims 1 and 3, **characterized in that** the drug is designed for treating acute infections especially when the organs concerned are from the ENT, pulmonary, ophthalmological, intestinal, urinary sphere, in particular when these infections start and even more especially when the infection is an ear infection or another respiratory infection starting with a nasopharyngitis.

6. Use according to claims 1 and 3, **characterized in that** the drug is designed for treating chronic infections especially when the organs concerned are from the ENT, pulmonary, intestinal, genitourinary spheres and even more especially when the infection is a chronic ear infection.
